# EUROPÄISCHE PATENTANMELDUNG

(11) **EP 2 919 007 A1**
(43) Veröffentlichungstag der Anmeldung: **16.09.2015**
(21) Anmeldenummer: 14159958.9
(22) Anmeldetag: 14.03.2014
(51) Int. Cl.: G01N 33/38

(54) **Feuchtesensor und Verfahren zur Messung des Trocknungsverlaufs einer statisch tragenden Gebäudestruktur**

(71) Anmelder: Rupprecht, Holger, 79183 Waldkirch (DE)
(72) Erfinder: Rupprecht, Holger, 79183 Waldkirch (DE)
(74) Vertreter: Fischer, Michael

(57) **Zusammenfassung**

Der vorliegenden Erfindung liegt die Aufgabe zugrunde, einen Feuchtesensor und ein Verfahren zur Messung des Trocknungsverlaufs einer statisch tragenden Gebäudestruktur anzugeben, mit denen es möglich ist, den Trocknungsverlauf einfach und kontinuierlich aufzeichnen und ebenso einfach auslesen zu können.

Bezüglich des Feuchtesensors wird diese Aufgabe gemäss der vorliegenden Erfindung durch einen Feuchtesensor (2) zur Messung des Trocknungsverlaufs einer statisch tragenden Gebäudestruktur (4), wie z.B. Betondecke, Estrich und dergleichen, gelöst, umfassend:
a) ein Gehäuse (8) mit einem mittels einer semipermeablen Membran (10) von der Gebäudestruktur (4) abgetrennten Messraum (12);
b) mindestens einen mit dem Messraum (12) assoziierten Sensor (14) zur Messung der relativen Luftfeuchtigkeit in dem Messraum (12);
c) einer im Gehäuse (8) angeordneten Datenverarbeitungseinheit (18) zur Verarbeitung und Speicherung der vom Sensor (14) gemessenen Werte;
d) eine Datenübertragungseinheit (20) zum berührungslosen Übertragen der gemessenen Werte; und
e) einen im Gehäuse (8) angeordneten Energiespeicher (16) zur Speisung des Sensors (14), der Datenverarbeitungseinheit (18) und der Datenübertragungseinheit (20).

Bezüglich des Verfahrens wird diese Aufgabe erfindungsgemäss durch ein Verfahren zur Messung des Trocknungsverlaufs einer statisch tragenden Gebäudestruktur (4), wie z.B. Betondecke, Estrich und dergleichen, gelöst, welches die folgenden Schritte umfasst:
a) Erstellen der Gebäudestruktur (4) durch Einbringen einer fliessfähigen aushärtbaren Mischung, zum Beispiel Zementmischung, in eine die zukünftige Gebäudestruktur umgebende Tragstruktur;
b) Anordnen eines Feuchtesensors (2), insbesondere eines Feuchtesensors gemäss einem der Ansprüche 1 bis 6, in der Gebäudestruktur (4);
c) Drahtloses Auslesen von mindestens einem mittels des Feuchtesensors (2) gemessenen Datensatzes durch ein mobiles Kommunikationsgerät (28); und
d) Auswerten der ausgelesenen Datensätze auf dem Kommunikationsgerät (28) oder auf einem mit dem Kommunikationsgerät assoziierbaren Auswerteeinheit.

## Beschreibung

Die vorliegende Erfindung bezieht sich auf einen Feuchtesensor und ein Verfahren zur Messung des Trocknungsverlaufs einer statisch tragenden Gebäudestruktur, wie z.B. Betondecke, Estrich und dergleichen.

In der Konstruktion von neuen Häusern, Werkhallen und in der Altbausanierung besteht ein hoher Bedarf an statisch einwandfreien Verbindungen von Holzbalken/Holzrahmen mit Betonelementen. Derartige Betonelemente werden mit den Holzbalken/Holzrahmen zu sogenannten Holz/Beton-Verbundtragwerken zusammengefügt und werden beispielsweise als Wände, Tragdecken oder (Fertig-)Dachelemente eingesetzt. Diese Holz/Beton-Verbundtragwerke haben gegenüber dem reinen Holzbau den Vorteil, dass sie einerseits trotz des Betonanteils dennoch vergleichsweise leicht sind und andererseits trotz des Holzanteils dennoch eine genügend hohe Wärmespeichermasse aufweisen. Weiter verbessern derartige Tragwerke die akustischen Eigenschaften eines Gebäudes entscheidend und haben ebenfalls eine gute Feuerwiderstandsdauer. Mit dem verbesserten Schallschutzverhalten verringern sie zudem spürbare Schwingungen von Böden und steifen somit das Gebäude statisch hervorragend aus. Abgesehen von diesen technischen Vorteilen befriedigen die sichtbaren Holzbalken oft auch die ästhetischen Wünsche der Bauherrschaft.

Zur Verbindung von einzelnen Holz/Beton-Verbundelementen zu diesen Holz/Beton-Verbundtragwerken sind verschiedene mehr oder weniger aufwendige Lösungen bekannt. Eine Lösung sieht beispielsweise die Ausbildung von Ortbeton-Taschen vor, die allerdings auf einer ansonsten im Holzbau eher betonfreien Trockenbaustelle vor Ort ausbetoniert werden müssen. Die Verwendung von Beton ist auf einer derartigen Trockenbaustelle in der Regel allerdings unerwünscht und immer mit einem vergleichsweise hohen Aufwand verbunden. Zusätzlich werden hier gekreuzt angeordnete Doppelgewindeschrauben verwendet. Diese von unten angesetzten Schrauben sind jedoch sichtbar, was den ästhetischen Eindruck dieser Verbundtragwerke schmälern kann. Grundsätzlich ist es auch möglich, Stahlteile in die Betonplatten einzulegen, welche nach der Montage der Verbundelemente verschweisst werden müssen. Allerdings ist im Holzbau die Brandgefahr beim Schweissen relativ hoch.

Weiter ist es bekannt, Beton- oder Zementschichten auf eine balkenartige Holztragstruktur aufzugiessen. Für die Verbindung der Holzbalken mit der Betonschicht werden Verschraubungen aller Art sowie Betonstopfen verwendet, die in in die Tragbalken gebohrte Löcher eingesetzt werden. Aufgrund der statisch nicht ausschaltbaren Relativbewegung von Betonschicht zu den Holzbalken werden diese Verschraubungen und Betonstopfen jedoch hohen Scherkräften ausgesetzt, die dazu führen können, dass die Verschraubung reisst und die Betonstopfen brechen.

Unter dem Strich ist daher bei derartigen gegossenen Schichten auch ein hohes Augenmerk auf die Einstellung eines geeigneten Trocknungsverlaufes zu legen. Trocknet beispielsweise eine gegossene Estrich-Schicht zu schnell, kann dies zu Aufwölbungen an den Rändern und zu Spannungsrissen im Innern der Estrichschicht führen, was die Estrich-Schicht minderwertiger werden lässt und zu Regressansprüchen gegen den Bauunternehmen führen könnte.

Oft trägt aber der Bauunternehmer an diesen Sachmängeln keine Schuld, weil er die übrigen an der Erstellung des Bauwerks beteiligten Personen darüber aufgeklärt hat, in welcher Weise die Austrocknung einer gegossenen Beton- oder Zementschicht vor sich zu gehen hat. In der Regel beschränken sich diese Randbedingungen bei der Trocknung darauf, dass weder zu stark gelüftet noch zu stark geheizt wird. Ebenso sollten aber auch zu kalte Bedingungen, und im Besonderen Frost, vermieden werden.

Kommt es nun dennoch zu Baumängeln, obliegt es Bauunternehmer nachzuweisen, dass er die Bedingungen korrekt eingehalten hat. Dieser Nachweis ist jedoch nur durch eine Messung des Feuchte- und Temperaturverlaufs der gegossenen Beton-/Zement-Schicht führbar. Geeignete Vorrichtungen zur Ausführung dieser "klimatischen" Überwachung der austrocknenden Schicht sind aber derzeit nicht verfügbar.

Der vorliegenden Erfindung liegt daher die Aufgabe zugrunde, einen Feuchtesensor und ein Verfahren zur Messung des Trocknungsverlaufs einer statisch tragenden Gebäudestruktur anzugeben, mit denen es möglich, den Trocknungsverlauf einfach und kontinuierlich aufzeichnen und ebenso einfach auslesen zu können.

Bezüglich des Feuchtesensors wird diese Aufgabe gemäss der vorliegenden Erfindung durch einen Feuchtesensor zur Messung des Trocknungsverlaufs einer statisch tragenden Gebäudestruktur, wie z.B. Betondecke, Estrich und dergleichen, gelöst, umfassend:
a) ein Gehäuse mit einem mittels einer semipermeablen Membran von der Gebäudestruktur abgetrennten Messraum;
b) mindestens einen mit dem Messraum assoziierten Sensor zur Messung der relativen Luftfeuchtigkeit in dem Messraum;
c) einer im Gehäuse angeordneten Datenverarbeitungseinheit zur Verarbeitung und Speicherung der vom Sensor gemessenen Werte;
d) eine Datenübertragungseinheit zum berührungslosen Übertragen der gemessenen Werte; und
einen im Gehäuse angeordneten Energiespeicher zur Speisung des Sensors, der Datenverarbeitungseinheit und der Datenübertragungseinheit.

Bezüglich des Verfahrens wird diese Aufgabe erfindungsgemäss durch ein Verfahren zur Messung des Trocknungsverlaufs einer statisch tragenden Gebäudestruktur, wie z.B. Betondecke, Estrich und dergleichen, gelöst, welches die folgenden Schritte umfasst:
a) Erstellen der Gebäudestruktur durch Einbringen einer fliessfähigen aushärtbaren Mischung, zum Beispiel Zementmischung, in eine die zukünftige Gebäudestruktur umgebende Tragstruktur;
b) Anordnen eines Feuchtesensors, insbesondere eines Feuchtesensors gemäss einem der Ansprüche 1 bis 6, in der Gebäudestruktur;
c) Drahtloses Auslesen von mindestens einem mittels des Feuchtesensors gemessenen Datensatzes durch ein mobiles Kommunikationsgerät; und
d) Auswerten der ausgelesenen Datensätze auf dem Kommunikationsgerät oder auf einem mit dem Kommunikationsgerät assoziierbaren Auswerteeinheit.

Auf diese Weise ermöglichen es der Feuchtesensor und das Verfahren zumindest den Feuchtegrad der Gebäudestruktur permanent oder periodisch zu messen und aufzuzeichnen. Mit einem entsprechenden mobilen Kommunikationsgerät können diese Datensätze dann drahtlos ausgelesen und entsprechend weiterverarbeitet werden. Hierzu kann beispielsweise auf dem mobilen Kommunikationsgerät eine Applikation ausgeführt werden, die das Auslesen der Datensätze durchführt, wenn sich das mobile Kommunikationsgerät in Funkreichweite des Feuchtesensors befindet. Auf diese Weise lässt sich auch der Energiebedarf auf dem Feuchtesensor auf ein vernünftiges Mass begrenzen, weil der Feuchtesensor die Datensätze nur über eine vergleichsweise geringe Reichweite aussenden muss, wie z.B. über einige Meter.

In einer vorteilhaften Weiterbildung der vorliegenden Erfindung kann der Sensor zusätzlich die Temperatur und/oder den Dampfdruck und/oder den Taupunkt in dem Messraum erfassen. Auf diese Weise können noch detailliertere Informationen über den Trocknungsverlauf der Gebäudestruktur gewonnen werden.

Wenn die Datenverarbeitungseinheit mit einem ID-Code programmierbar ist, kann zusätzlich auch noch eine genaue Lokalisierung der einzelnen Feuchtesensoren vorgenommen werden, was beispielsweise ratsam ist, wenn in der Gebäudestruktur eine Vielzahl von Feuchtesensoren angeordnet sind.

Eine weiter vorteilhafte Vorgehensweise für die spätere Auswertung der gemessenen Daten kann es vorsehen, dass die Datenverarbeitungseinheit zu den mittels des Sensor gemessenen Werten einen Messzeitpunkt und/oder den ID-Code in einem Datensatz abspeichert und diesen Datensatz zur Übertragung durch die Datenübertragungseinheit bereitstellt. So ist beispielsweise der zeitliche und räumliche Verlauf der Trocknung gemäss der Anordnung der Feuchtesensoren nachvollziehbar.

Zum einfachen Auslesen der Datensätze kann es vorgesehen sein, dass die Übertragung der gemessenen Werte durch die Datenübertragungseinheit durch ein externes Gerät drahtlos triggerbar ist. Beispielhaft kann hierfür ein mit einer entsprechenden Applikation ausgestattetes Smartphone genannt sein, dass mit einem Trigger-Signal bei entsprechend naher räumlicher Annäherung des Smartphones an einen Feuchtesensor das Aussenden der Datensätze durch den Feuchtesensor resp. durch seine Datenübertragungseinheit erfolgt.

Da der Energievorrat des Energiespeichers nur begrenzt ist und der Feuchtesensor ja in der Gebäudestruktur eingelassen ist, kann es zur Aufrechterhaltung der Funktion des Feuchtesensors vorgesehen sein, dass der Energiespeicher über von aussen zugänglich am Gehäuse angeordnete Ladekontakte verfügt und somit auch von aussen selbst im eingebauten Zustand wieder aufgeladen werden kann.

In verfahrensmässig vorteilhafter Weise kann der Feuchtesensor bereits vor dem Einbringen der Zementmischung an einer definierten Position relativ zur Tragstruktur angebracht wird. So kann er beispielsweise an einem Holzbalken befestigt und später zumindest so mit eingegossenen werden, dass sich der mit der der semipermeable Membran abgetrennte Messraum im Feuchteaustausch mit der umgebenden aushärtenden Schicht (der späteren Gebäudestruktur) befindet. Alternativ kann der Feuchtesensor auch in ein in die aushärtende Zementmischung eingefügtes Bohrloch eingesetzt sein.

Zur Auslesung der Daten kann es in einfacher und besonders bedienerfreundlichen Weise vorgesehen sein, dass das Auslesen des Datensatzes berührungslos durch eine Triggerung mittels einer auf dem mobilen Kommunikationsgerät ausgeführten Applikation vorgenommen wird. Dabei kann die Übertragung des Datensatzes mittels Bluetooth oder einem ähnlichen Datenübertragungsstandard kurzer Reichweite erfolgen.

Alternativ hierzu kann der Feuchtesensor auch selbst eine Mobiltelefonfunktionalität aufweisen, sodass die Übertragung des Datensatzes mittels eines vom Feuchtesensor ausgesendeten SMS erfolgt. Die Empfangsstation kann dabei wieder das mobile Kommunikationsgerät sein, dass mittels der auf ihm ausgeführten Applikation auch direkt eine Auswertung der Datensätze vornehmen kann. Alternativ können die SMS aber auch an Meldezentrale des Bauunternehmens gesendet werden. Beide Lösungen erlauben es aber den gemessenen Verlauf mit vorgebbaren Soll-Trocknungsverläufen zu vergleichen und bei kritischen Abweichung auch entsprechende Alarmmitteilungen auszulösen.

Besonders bevorzugte Ausführungsbeispiele der vorliegenden Erfindung werden anhand einer Zeichnung näher erläutert. Dabei zeigen:
- Figur 1: schematisch einen Längsschnitt durch einen Feuchtesensor; und
- Figur 2: einen Estrichboten mit mehreren Feuchtesensoren gemäss Figur 1 und einem schematischen Ablauf des Erfassungsverfahrens.

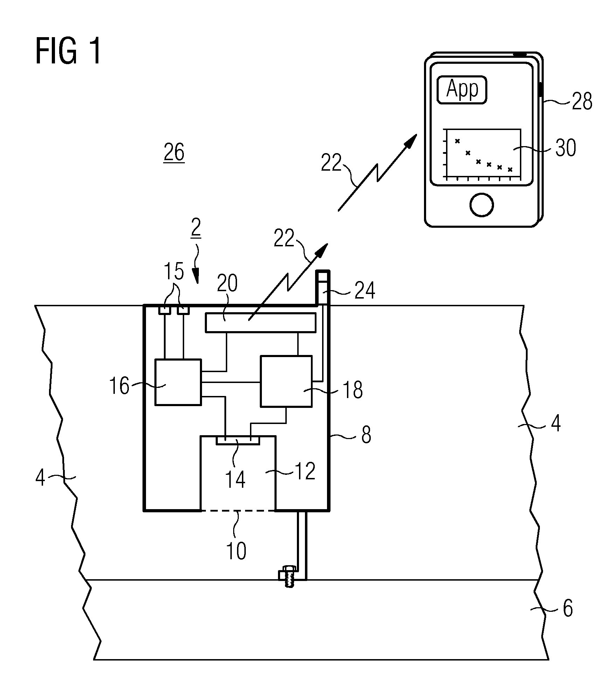

Figur 1 zeigt schematisch einen Längsschnitt durch einen Feuchtesensor 2 zur Messung des Trocknungsverlaufs einer statisch tragenden Gebäudestruktur, hier einer Betondecke 4. Die Betondecke 4 wird zudem von Holzbalken 6 getragen. Der Feuchtesensor 2 umfasst ein Gehäuse 8 mit einem mittels einer semipermeablen Membran 10 von der Betondecke 4 abgetrennten Messraum 12. Der Feuchtesensor 2 ist dabei vor der Einbringung der Betondecke 4 mit dem Holzbalken verschraubt und in seiner Höhe über dem Holzbalken so justiert worden, dass die Oberkante des Gehäuses 8 mit der Oberkante der später eingegossenen Betondecke 4 abschliesst. Alternativ könnte der Feuchtesensor 2 auch später in die gegossene Betondecke 4 eingesetzt werden, z.B. in ein in die vorzugsweise noch nicht ausgehärtete Betondecke 4 eingebrachtes Topfloch.

Mit dem Messraum 12 ist ein Sensor 14 assoziiert, der die relative Luftfeuchtigkeit und die Temperatur in dem Messraum 12 misst. Der Sensor 14 ist zu seiner Spannungsversorgung mit einer in dem Gehäuse 8 angeordneten Spannungsversorgungseinheit 16 verbunden. In dem Gehäuse 8 ist weiter eine Datenverarbeitungseinheit 18 angeordnet, die zur Verarbeitung und Speicherung der vom Sensor 14 gemessenen Werte dient. Die Datenverarbeitungseinheit 18 stellt auch die gemessenen Werte unter Hinzufügung eines Zeitstempels des Messzeitpunkts zur berührungslosen Übertragung der Werte mittels einer Datenübertragungseinheit 20 bereit, was durch Pfeile 22 symbolisiert sein soll.

Im vorliegenden Ausführungsbeispiel weist der Feuchtsensor 2 zudem einen oberhalb der Betondecke 4 liegenden zweiten Messraum 24 auf, um zusätzlich die klimatischen Verhältnisse im über der Betondecke 4 liegenden Luftraum 26 zu erfassen und an die Datenverarbeitungseinheit 18 zu melden. Vorliegend versorgt die Datenverarbeitungseinheit 18, die selbst mit der Spannungsversorgungseinheit 16 verbunden ist, den mit dem zweiten Messraum assoziierten und hier aber aus Platzgründen nicht weiter dargestellten zweiten Sensor mit der erforderlichen elektrischen Leistung. Der Spannungsversorgungseinheit 16, z.B. ein Akku, weist zudem von aussen zugänglich am Gehäuse 8 angeordnete Ladekontakte 15 auf.

Zusätzlich können die Sensoren 14 auch noch den Dampfdruck und/oder den Taupunkt in den Messräumen 12, 24 erfassen. Dies ist aber ebenso wie der zweite Messraum 26 optional.

In der schematischen Darstellung gemäss Figur 1 ist auch ein Smartphone 28 erkennbar, welches mittels einer Applikation App die von dem Feuchtesensor 2 ausgesendeten Werte empfängt, speichert und auswertet, was durch ein im Display des Smartphones 28 dargestelltes Chart 30 symbolisiert sein soll. So kann die Applikation App weiter dazu dienen, die Datenverarbeitungseinheit 18 mit einem ID-Code programmierbar ist. Die Datenverarbeitungseinheit 18 kann so zu den mittels des Sensors 14 gemessenen Werten einen Messzeitpunkt und den ID-Code in einem Datensatz abspeichert und diesen Datensatz zur Übertragung durch die Datenübertragungseinheit 20 bereitstellen. Die Übertragung der gemessenen Werte durch die Datenübertragungseinheit 20 kann dann beispielsweise auch durch das Smartphone 28 drahtlos, z.B. mit Bluetooth, getriggert werden.

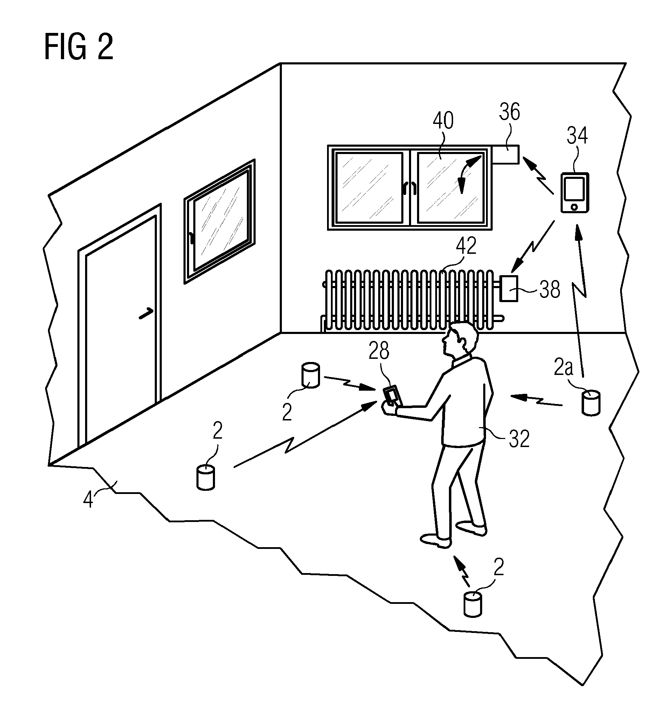

Das Verfahren zur Messung des Trocknungsverlaufs der statisch tragenden Betondecke 4 ist daher vergleichsweise ausführbar. Den Start stellt das Erstellen der Betondecke 4 durch Einbringen einer fliessfähigen aushärtbaren Zementmischung in eine die zukünftige Betondecke 4 umgebende Tragstruktur, wie z.B. die hier dargestellten Holzbalken 6 sowie die hier nicht weiter dargestellten Schalbretter. Die Anordnung des vorstehend beschriebenen Feuchtesensors 2 kann wie vorstehend ebenso schon beschrieben vor oder nach dem Eingiessen der Betondecke vorgesehen werden. Die von dem Feuchtesensor 2 gemessenen Werte werden drahtlos auf dem Smartphone 28 empfangen und ausgewertet. Zum Empfang kann es vorgesehen sein, dass eine Bedienperson 32 - so wie dies in Figur 2 dargestellt ist, den Raum durchschreitet und sich jeweils in den Sendebereich der Feuchtesensoren 2 begibt, um die von den Feuchtesensoren ausgesendeten Datensätze zu empfangen. Die Übertragung der Datensätze kann dabei mittels Bluetooth oder einem ähnlichen Datenübertragungsstandard kurzer Reichweite erfolgen. Die Übertragung der Datensätze könnte aber auch mittels einer vom Feuchtesensor 2 ausgesendeten SMS erfolgen, wozu die Datenübertragungseinheit 20 dann aber auch eine Mobilfunkfunktionalität aufweisen müsste.

Die empfangenen Datensätze können dann auf dem Smartphone 28 selbst oder alternativ auch einer mit Smartphone 28 gekoppelten Auswerteeinheit ausgewertet werden. Auf diese Weise ermöglichen es der Feuchtesensor 2 und das Verfahren zumindest den Feuchtegrad der Betondecke 4 permanent oder periodisch zu messen und aufzuzeichnen. Mit dem entsprechenden Smartphone 28 können diese Datensätze dann drahtlos ausgelesen und entsprechend weiterverarbeitet werden. Hierzu kann beispielsweise auf dem Smartphone 28 die Applikation App ausgeführt werden, die das Auslesen der Datensätze durchführt, wenn sich das Smartphone 28 in Funkreichweite des Feuchtesensors 2 befindet. Auf diese Weise lässt sich auch der Energiebedarf auf dem Feuchtesensor 2 auf ein vernünftiges Mass begrenzen, weil der Feuchtesensor 2 die Datensätze nur über eine vergleichsweise geringe Reichweite aussenden muss, wie z.B. über einige Meter.

In Figur 2 ist für einen Feuchtesensor 2a noch eine kleine Anpassung des Verfahrens für den Fall gezeigt, dass die Datensätze der Feuchtesensoren 2, 2a von einem ortsfest installierten Steuerungsmodul 34 empfangen werden. Das Steuerungsmodul 34 kann diese Datensätze dann auswerten und beispielsweise zu dem Ergebnis kommen, dass die Raumtemperatur und die Luftfeuchtigkeit im Luftraum zu hoch für die ordnungsgemässe Austrocknung der Betondecke 4 sind. Das Steuerungsmodul 34 kann dann wiederum ein Fenstersteuerungsmodul 36 und ein Heizungssteuerungsmodul 38 anfunken, welche dann ein Fenster 40 öffnen bzw. eine Heizung 42 abschalten. Alternativ könnte es aber auch vorgesehen sein, dass das Smartphone 28 die empfangenen Datensätze an das Steuerungsmodul 34 sendet oder diese Steuerungsaufgabe sogar selbst übernimmt und das Fenster- und Heizungssteuerungsmodul 36 bzw. 38 selbst anfunkt um die gewünschte Anpassung des Raumklimas zum ordnungsgemässen Austrocknen der Betondecke 4 vorzunehmen.

## Patentansprüche

1. Feuchtesensor zur Messung des Trocknungsverlaufs einer statisch tragenden Gebäudestruktur, wie z.B. Betondecke, Estrich, Wand und dergleichen, umfassend:
a) ein Gehäuse mit einem mittels einer semipermeablen Membran von der Gebäudestruktur abgetrennten Messraum;
b) mindestens einen mit dem Messraum assoziierten Sensor zur Messung der relativen Luftfeuchtigkeit in dem Messraum;
c) einer im Gehäuse angeordneten Datenverarbeitungseinheit zur Verarbeitung und Speicherung der vom Sensor gemessenen Werte;
d) eine Datenübertragungseinheit zum berührungslosen Übertragen der gemessenen Werte; und
e) einen im Gehäuse angeordneten Energiespeicher zur Speisung des Sensors, der Datenverarbeitungseinheit und der Datenübertragungseinheit.

2. Feuchtesensor nach Anspruch 1,
**dadurch gekennzeichnet, dass**
der Sensor zusätzlich die Temperatur und/oder den Dampfdruck und/oder den Taupunkt in dem Messraum erfasst.

3. Feuchtesensor nach Anspruch 1 oder 2,
**dadurch gekennzeichnet, dass**
die Datenverarbeitungseinheit mit einem ID-Code programmierbar ist.

4. Feuchtesensor nach einem der vorangehenden Ansprüche,
**dadurch gekennzeichnet, dass**
die Datenverarbeitungseinheit zu den mittels des Sensor gemessenen Werten einen Messzeitpunkt und/oder den ID-Code in einem Datensatz abspeichert und diesen Datensatz zur Übertragung durch die Datenübertragungseinheit bereitstellt.

5. Feuchtesensor nach einem der vorangehenden Ansprüche,
**dadurch gekennzeichnet, dass**
die Übertragung der gemessenen Werte durch die Datenübertragungseinheit durch ein externes Gerät drahtlos triggerbar ist.

6. Feuchtesensor nach einem der vorangehenden Ansprüche,
**dadurch gekennzeichnet, dass**
der Energiespeicher über von aussen zugänglich am Gehäuse angeordnete Ladekontakte verfügt.

7. Verfahren zur Messung des Trocknungsverlaufs einer statisch tragenden Gebäudestruktur, wie z.B. Betondecke, Estrich, Wand und dergleichen, umfassend die folgenden Schritte:
a) Erstellen der Gebäudestruktur durch Einbringen einer fliessfähigen aushärtbaren Mischung, zum Beispiel einer Zementmischung, in eine die zukünftige Gebäudestruktur umgebende Tragstruktur;
b) Anordnen eines Feuchtesensors, insbesondere gemäss einem der Ansprüche 1 bis 6, in der Gebäudestruktur;
c) Drahtloses Auslesen von mindestens einem mittels des Feuchtesensors gemessenen Datensatzes durch ein mobiles Kommunikationsgerät; und
d) Auswerten der ausgelesenen Datensätze auf dem Kommunikationsgerät oder auf einem mit dem Kommunikationsgerät assoziierbaren Auswerteeinheit.

8. Verfahren nach Anspruch 7,
**dadurch gekennzeichnet, dass**
der Feuchtesensor bereits vor dem Einbringen der Zementmischung an einer definierten Position relativ zur Tragstruktur angebracht wird.

9. Verfahren nach Anspruch 7,
**dadurch gekennzeichnet, dass**
der Feuchtesensor in ein in die aushärtende Zementmischung eingefügtes Bohrloch eingesetzt wird.

10. Verfahren nach einem der Ansprüche 7 bis 9,
**dadurch gekennzeichnet, dass**
das Auslesen des Datensatzes berührungslos durch eine Triggerung mittels einer auf dem mobilen Kommunikationsgerät ausgeführten Applikation vorgenommen wird.

11. Verfahren nach Anspruch 10,
**dadurch gekennzeichnet, dass**
die Übertragung des Datensatzes mittels Bluetooth oder einem ähnliche Datenübertragungsstandard kurzer Reichweite erfolgt.

12. Verfahren nach einem der Ansprüche 7 bis 9,
**dadurch gekennzeichnet, dass**
die Übertragung des Datensatzes mittels eines vom Feuchtesensor ausgesendeten SMS erfolgt.
